Europäisches Patentamt

European Patent Office

Office européen des brevets

(11) **EP 1 085 316 A2**

(19)

(12) **EUROPEAN PATENT APPLICATION**

(43) Date of publication:
**21.03.2001 Bulletin 2001/12**

(51) Int Cl.⁷: **G01N 27/22**, G01N 33/487,
G01N 27/02

(21) Application number: **00307950.6**

(22) Date of filing: **13.09.2000**

(84) Designated Contracting States:
**AT BE CH CY DE DK ES FI FR GB GR IE IT LI LU
MC NL PT SE**
Designated Extension States:
**AL LT LV MK RO SI**

(30) Priority: **15.09.1999 GB 9921682**

(71) Applicant: **ABER INSTRUMENTS LIMITED
Cefnllan, Aberystwyth SY23 3AH (GB)**

(72) Inventor: **Todd, Robert William Dr.
Powys, SY20 8QC, (GB)**

(74) Representative: **Gibson, Stewart Harry
URQUHART-DYKES & LORD,
Three Trinity Court,
21-27 Newport Road
Cardiff CF24 0AA (GB)**

(54) **Detecting cell-condition changes**

(57)     Changes in the parameters or conditions of cells in a suspension are determined by making impedance measurements of the suspension at a number of different frequencies, these measurements being used to determine any change in the frequency-dependent impedance characteristic, indicative of changes other than cell concentration or conductivity of the medium in which the cells are suspended.

Figure 2

EP 1 085 316 A2

**Description**

FIELD OF THE INVENTION

The present invention relates to a method of and apparatus for detecting changes in the condition of cells in a suspension.

BACKGROUND OF THE INVENTION

There are various processes in biotechnology industries, notably including fermentation processes, where there is a need to monitor the population of live cells in a suspension. US patent No. 4,965,206 (European patent No. 0,282,532) discloses and claims an on-line method of determining biomass in a suspension, in which electrical capacitance measurements are made, between electrodes spaced apart within the suspension. The electrical impedance of a live cell suspension exhibits a characteristic which is dependent on frequency. In the method of US patent No. 4,965,206, a predetermined operating frequency is selected, this frequency being one at which, for the particular suspension in question, the dielectric permittivity of the material in the bulk of the suspension depends predominantly on the so-called β-dispersion, and so varies more or less solely in dependence upon the volume fraction of the medium enclosed by the cytoplasmic membranes of the cells. The electrical capacitance measurement thus indicates the biovolume, from which the biomass can be determined.

The instrument used for performing the above method is calibrated against a reference method. Usually the measurements made on the calibrated instrument are performed at a single operating frequency, sometimes with measurements being made at a second or third frequency to allow correction for gas hold-up and/or electrode artefacts.

Over the range of frequencies in which the dielectric permittivity of the material in the bulk of the suspension depends predominantly the β-dispersion, the frequency-dependence of the capacitance (the real part of the complex permittivity) of the suspension is generally of a form defined by the following equations:

$$\Delta C_f \propto PRC_m \frac{1 + (f/f_c)^{1-\alpha} \sin 90\alpha}{1 + 2(f/f_c)^{1-\alpha} \sin 90\alpha + (f/f_c)^{2-2\alpha}} \qquad (1)$$

and

$$f_c = \frac{1}{RC_m(1/S_i + 1/2S_o)} \qquad (2)$$

where

$\Delta C_f$ = Capacitance above background at frequency $f$
$P$ = total volume fraction enclosed by cell membranes,
$f$ = measuring frequency,
$f_c$ = characteristic frequency,
$\alpha$ = Cole-Cole shape parameter,
$R$ = cell radius,
$C_m$ = membrane capacitance constant,
$S_i$ = cytoplasm conductivity,
$S_o$ = suspending medium conductivity.

Whilst the frequency-dependent capacitance characteristic is generally of the form defined by Equation 91), its precise form may depart slightly from this due to various effects or parameters of the cell suspension: these may include cell size, shape and structure, cell membrane properties, cytoplasm conductivity, and the statistics of the non-homogenous cell population.

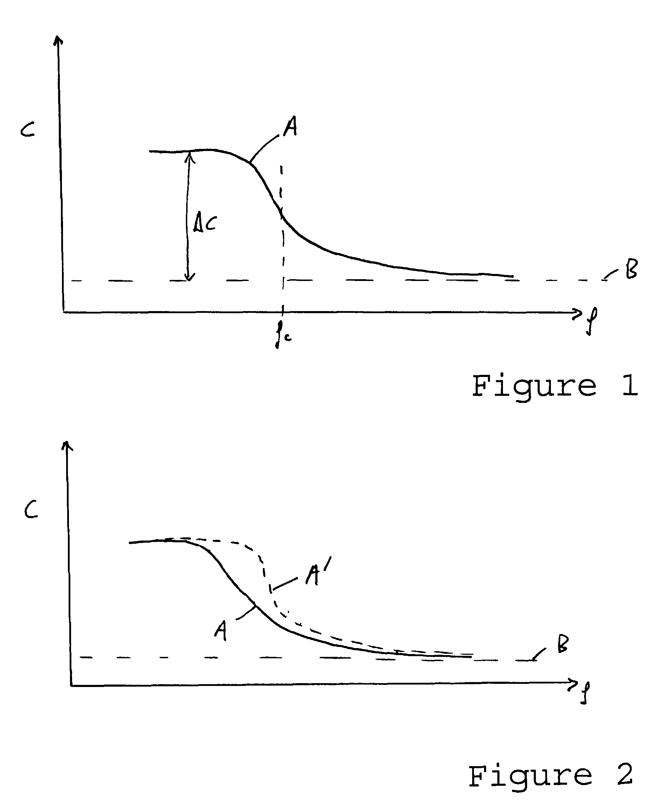

Equation (1) defines a curve, as shown in Figure 1, approximately the shape of an inverted sigmoid, having a plateau over a relatively low range of frequencies, then a curved transition down towards a lower level at high frequencies, which tends towards a baseline or background level corresponding to the permittivity of the liquid in which the cells are suspended. The β-dispersion, or increase in permittivity above the baseline level, is an effect caused mainly by the presence of relatively ion-impermeable membranes of the live cells: the β-dispersion is therefore a property of live cells and its magnitude is proportional to the volume fraction of the cells within the suspension. There is a similar (but inverted) sigmoid curve in the conductivity vs. frequency characteristic.

It will be noted from the above Equations that the relationship depends on the conductivity of the suspending medium. Accordingly, in instruments marketed by us, in accordance with US patent No. 4,965,206, the conductivity is measured and used by the instrument software to effect a correction of the measured capacitance values. Even so, the single-frequency measurement of capacitance is unable to distinguish between a change in the concentration of viable cells, and changes in other parameters or conditions of the cells (e.g. the cell size).

SUMMARY OF THE INVENTION

We have now devised a method of and apparatus for detecting changes in the parameters or conditions of cells in a suspension, other than changes in cell concentration or the conductivity of the suspending medium.

In accordance with the present invention, there is provided a method of detecting changes in the parameters or conditions of cells in a suspension, the method comprising the steps of making impedance measurements of the suspension, at a number of different frequencies, and using said measurements to detect any change in the frequency-dependent impedance characteristic, indicative of changes other than cell concentration or conductivity of the medium in which the cells are suspended.

Thus, a change in cell concentration acts as a scale multiplier to a characteristic curve of the form of Equation (1), without changing the fundamental shape of the curve, and the medium conductivity affects the curve in a predictable manner. However, changes in the fundamental shape of the characteristic curve occur, and/or a shift along the frequency axis occurs, in the event of a change of any of a number of other parameters or conditions of the cells, particularly cell size, shape and structure, cell membrane properties, cytoplasm conductivity, and the statistics of the non-homogenous cell population.

Accordingly, by monitoring for any change in a frequency-dependent impedance characteristic, which is not due to concentration or medium conductivity changes, a change in any other parameter or condition can be detected. The suspension can then be examined to determine the cause.

Initially, the frequency dependence of one or more calibration samples is determined as a function of cell concentration and conductivity of the medium. Thereafter, the method of the present invention monitors for any change in the frequency-dependent characteristic. In particular, the method monitors for any change in the fundamental shape (normalised shape) of the curve, or any shift along the frequency axis (i.e. change in the characteristic frequency).

The method may utilise the frequency-dependent capacitance characteristic, or the frequency-dependent conductivity characteristic: alternatively, it may monitor both of these characteristics.

Also in accordance with the present invention, there is provided an apparatus for detecting changes in the parameters or conditions of cells in a suspension, the apparatus comprising means for making impedance measurements of the suspension, at a number of different frequencies, and means for using said measurements to determine any change in the frequency-dependent impedance characteristic indicative of changes other than cell concentration or conductivity of the medium in which the cells are suspended.

BRIEF DESCRIPTION OF THE DRAWINGS

An embodiment of the present invention will now be described by way of example only and with reference to the accompanying drawings, in which:

FIGURE 1 shown a typical frequency-dependent capacitance characteristic for a biological cell suspension; FIGURE 2 shows schematically changes which can occur in the shape of the characteristic curve;
FIGURE 3 is a schematic diagram of an apparatus in accordance with the present invention; and
FIGURE 4 is a schematic block diagram of the measurement and processing unit of the apparatus shown in Figure 3.

DETAILED DESCRIPTION OF THE PREFERRED EMBODIMENT

Referring to Figure 1, there is shown a typical frequency-dependent capacitance characteristic curve A for a biological cell suspension, over the range of frequencies in which the β-dispersion predominates. Thus, over a range of frequencies, the value of the capacitance, relative to the baseline or background level B, is predominantly due to the β-dispersion effect: the capacitance is directly proportioned to the dielectric permittivity of the material in the bulk of the suspension. Changes in cell concentration act as a scale multiplier on the characteristic curve A, without changing the fundamental shape of this curve.

As indicated at A' in Figure 2, a number of other changes in the parameters or condition of the cells lead to a change in the fundamental shape of the characteristic curve, and/or a shift along the frequency axis. Thus, by detecting a change in the curve, other than changes due to changes in cell concentration or medium conductivity, an indication is given that there has been a change in at least one of the other parameters or conditions: a sample of the suspension can then be taken in order to investigate the cause of the change.

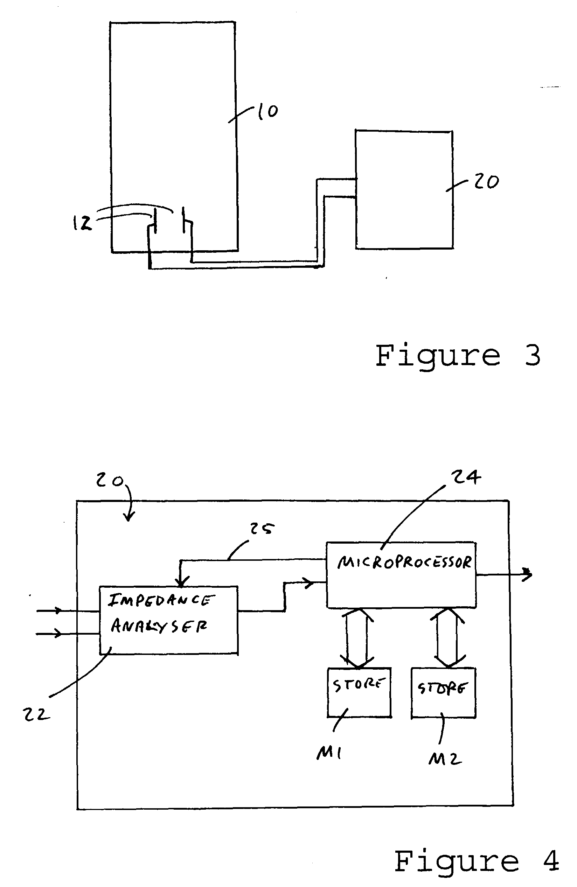

Figure 3 shows, in schematic form, an apparatus in accordance with the invention. A fermentation vessel 10 contains the suspension and is provided with electrodes 12 spaced apart in the suspension, which are connected to a measurement and processing unit 20. The unit 20 serves to make capacitance and conductivity measurements, at desired intervals of time and at a predetermined frequency, in order to determine the cell concentration, in particular the biovolume and/or biomass, in accordance with the principles of US patent No. 4,965,206. Whilst the conductivity measured is the conductivity of the suspension, this is corrected to determine the conductivity of the medium itself. In addition, the unit 20 serves to make capacitance and conductivity measurements, at desired intervals of time, at a number of different frequencies, then analyses these measurements to determine if any change in the frequency-dependent characteristic curve has occurred, since calibration with one or more reference suspensions, indicative of some change other than in cell concentration or medium conductivity.

Referring to Figure 4, the unit 20 includes an impedance analyser 22 for making capacitance and/or conductivity measurements. The operating frequency is controlled by a microprocessor 24 over a control line 25. In the calibration phase, data defining the frequency-dependent characteristic curve is stored in a memory M1. Then, in subsequent use of the apparatus, the impedance analyser 22 makes a series of impedance measurements at periodic intervals of time: each series of measurements includes capacitance or conductivity measurements made at different frequencies and these measurements, defining the frequency-dependent characteristic at that instant of time, are stored in a memory M2. The microprocessor 24 retrieves each new set of data, defining the current frequency-dependent curve, for comparison with the curve defined by the reference set of date held in memory M1. The microprocessor is programmed to detect any change in the fundamental shape of the curve (greater than a predetermined change) or any shift of the curve along the frequency axis (greater than a predetermined shift), to provide an indicator signal to alert the operating personnel: these personnel can then examine the suspension in order to determine the change.

Whilst Figure 3 shows the apparatus associated with a fermentation vessel, it may instead be associated with a pipe conveying the suspension, or with a storage tank, or with a sample chamber.

As previously indicated, the apparatus may be arranged to monitor for any change in the capacitance-frequency characteristic, or in the conductivity-frequency characteristic, or both.

The electrode system may be as described in US patent No. 4,810,650(European patent No. 0,281,602). Instead, capacitively-coupled or inductively-coupled electrodes may be used, for measurement of capacitance and/or conductivity of the medium.

**Claims**

1.   A method of detecting changes in the parameters or conditions of cells in a suspension, the method comprising the steps of making impedance measurements of the suspension, at a number of different frequencies, and using said measurements to detect any change in the frequency-dependent impedance characteristic, indicative of changes other than cell concentration or conductivity of the medium in which the cells are suspended.

2.   A method as claimed in claim 1 in which said measurements are used to detect any change in the fundamental shape of said frequency-dependent impedance characteristic.

3.   A method as claimed in claim 1, in which said measurements are used to detect any displacement, along the frequency axis, of said frequency-dependent impedance characteristic.

4.   A method as claimed in claim 1, in which said frequency-dependent impedance characteristic comprises a frequency-dependent capacitance characteristic.

5.   A method as claimed in claim 1, in which said frequency-dependent impedance characteristic comprises a frequency-dependent conductivity characteristic.

6.   An apparatus for detecting changes in the parameters or conditions of cells in a suspension, the apparatus comprising means for making impedance measurements of the suspension, at a number of different frequencies, and means for using said measurements to determine any change in the frequency-dependent impedance characteristic, indicative of changes other than cell concentration or conductivity of the medium in which the cells are suspended.

7. An apparatus as claimed in claim 6, comprising means for using said measurements to detect any change in the fundamental shape of said frequency-dependent impedance characteristic.

8. An apparatus as claimed in claim 6, comprising means for using said measurements to detect any displacement, along the frequency axis, of said frequency-dependent impedance characteristic.

9. An apparatus as claimed in claim 6, in which said means for making impedance measurements comprises means for making capacitance measurements.

10. An apparatus as claimed in claim 6, in which said means for making impedance measurements comprises means for making conductivity measurements.

Figure 1

Figure 2

Figure 3

Figure 4